# EUROPEAN PATENT APPLICATION

(11) **EP 4 249 596 A1**
(43) Date of publication of application: **27.09.2023**
(21) Application number: 21894579.8
(22) Date of filing: 15.11.2021
(51) Int. Cl.: C12N 15/24, A61K 35/768, A61K 38/19, A61K 38/20, C12N 7/01

(54) **NEW GENE RECOMBINANT VACCINIA VIRUS AND UTILIZATION THEREOF**

(30) Priority: 17.11.2020 JP 2020191128
(71) Applicant: National University Corporation Tottori University, Tottori-shi, Tottori 680-8550 (JP)
(72) Inventor: NAKAMURA Takafumi, Yonago-shi, Tottori 683-8503 (JP); WAKIMIZU Emi, Yonago-shi, Tottori 683-8503 (JP); NAKATAKE Motomu, Yonago-shi, Tottori 683-8503 (JP); KUROSAKI Hajime, Yonago-shi, Tottori 683-8503 (JP)
(74) Representative: Wohlfahrt, Jan Günther
(86) International application number: PCT/JP2021/041825
(87) International publication number: WO 2022/107705

(57) **Abstract**

The present invention provides a vaccinia virus into which a therapeutic gene has been introduced as an exogenous gene and a therapeutic composition comprising the same. The vaccinia virus comprises at least one immune regulating gene as an exogenous gene.

## Description

### Technical Field

The present invention relates to a vaccinia virus carrying a therapeutic gene.

### Background Art

Currently, preclinical and clinical studies on cancer treatment using five viruses are actively being conducted worldwide. This cancer virotherapy involves a method utilizing the inherent nature of viruses, which is to kill infected cells or tissues while growing and propagating therein, for cancer treatment. The method exhibits an anticancer effect through more diverse mechanisms, including firstly oncolysis through viral growth and secondly induction of antitumor immunity associated therewith, as compared with conventional radiotherapy and chemotherapy.

Vaccine strains of a vaccinia virus established in Japan and used as smallpox vaccines in humans are available and have been proved to be highly safe (refer to Non Patent Literature 1). However, these virus strains still showed weak growth in normal tissues. To establish a safer cancer virotherapy, improvement was therefore required so as to allow them to grow only in cancer cells. Accordingly, these vaccine strains were improved using gene recombination techniques, and recombinant vaccinia viruses that specifically grow in and kill cancer cells were successfully developed using abnormal regulation of the MAPK/ERK pathway in a wide range of cancers as an indicator (refer to Patent Literatures 1 and 2).

### Citation List

### Patent Literature

Patent Literature 1
   International Publication No. WO 2011/125469
Patent Literature 2
   International Publication No. WO 2015/076422

### Non Patent Literature

Non Patent Literature 1
Protein, Nucleic Acid and Enzyme, Vol.48, No. 12 (2003), pp.1693-1700

### Summary of Invention

### Technical Problem

An object of the present invention is to provide a vaccinia virus into which a therapeutic gene has been introduced as an exogenous gene, and a therapeutic composition comprising the vaccinia virus.

### Solution to Problem

While studying development of a vaccinia virus having a higher antitumor effect, the present inventors found that a synergistic anticancer effect was exhibited by introducing a gene encoding a protein capable of regulating immunity as a therapeutic gene into an oncolytic vaccinia virus, and thus accomplished the present invention.

That is, the present invention provides the following inventions:
[1] A vaccinia virus comprising at least one immune regulating gene as an exogenous gene.
[2] The vaccinia virus according to [1], which comprises two or three immune regulating genes as exogenous genes.
[3] The vaccinia virus according to [1] or [2], wherein the vaccinia virus is the LC16 strain, the LC16mO strain, or the LC16m8 strain modified so that the B5R gene is expressed.
[4] The vaccinia virus according to any of [1] to [3], which is deficient in functions of the K2L gene or the HA gene or the K2L gene and the HA gene and induces cell death by causing fusion of infected cells.
[5] The vaccinia virus according to any of [1] to [4], which is an oncolytic vaccinia virus.
[6] The vaccinia virus according to [4] or [5], which does not grow in a normal cell, but grows specifically in a cancer cell and has an oncolytic effect damaging a cancer cell specifically.
[7] The vaccinia virus according to any of [1] to [6], wherein the immune regulating gene is selected from the group consisting of the gene encoding IL-12, the gene encoding CCL21, the gene encoding IL-7, the gene encoding PDlscFv (scFv of anti-PD-1 antibody), and the gene encoding CD40L (CD154).
[8] The vaccinia virus according to any of [1] to [7], which comprises the gene encoding IL-12 and the gene encoding CCL21 in combination.
[9] The vaccinia virus according to any of [1] to [7], which comprises the gene encoding IL-12, the gene encoding CCL21, and the gene encoding IL-7 in combination.
[10] The vaccinia virus according to any of [1] to [7], which comprises any gene combination of the following (I) to (v):
   (I) a combination of the gene encoding IL-12 and the gene encoding IL-7;
   (ii) a combination of the gene encoding IL-12 and the gene encoding PD1scFv;
   (iii) a combination of the gene encoding IL-7 and the gene encoding CCL21;
   (iv) a combination of the gene encoding CD40L and the gene encoding IL-7; and
   (v) a combination of the gene encoding CD40L and the gene encoding CCL21.
[11] A pharmaceutical composition for cancer treatment which comprises the vaccinia virus according to any of [1] to [10].
[12] A composition comprising vaccinia viruses in combination, the vaccinia viruses being two or more types of the vaccinia virus according to any of [1] to [10] comprising one or two immune regulating genes as exogenous gene(s), wherein each of the vaccinia viruses comprises different immune regulating gene(s).
[13] The composition according to [12], which comprises in combination a vaccinia virus comprising a combination of the gene encoding IL-12 and the gene encoding IL-7 and a vaccinia virus comprising the gene encoding CCL21.
[14] The composition according to [12] or [13], which is a pharmaceutical composition for cancer treatment.
[15] A kit comprising vaccinia viruses in combination, the vaccinia viruses being two or more types of the vaccinia virus according to any of [1] to [10] comprising one or two immune regulating genes as exogenous gene(s), wherein each of the vaccinia viruses comprises different immune regulating gene(s).
[16] The kit according to [15], which comprises in combination a vaccinia virus comprising a combination of the gene encoding IL-12 and the gene encoding IL-7 and a vaccinia virus comprising the gene encoding CCL21.
[17] The kit according to [15] or [16], which is a kit for cancer treatment.

The present specification encompasses the content disclosed in JP Patent Application No. 2020-191128, which is the basis of the priority of the present application.

### Advantageous Effects of Invention

By introducing a gene encoding a protein capable of regulating immunity as a therapeutic gene into an oncolytic vaccinia virus, a higher anticancer effect is exhibited by a synergic effect of the antitumor effect of the vaccinia virus and the effect of the therapeutic gene.

### Brief Description of Drawings

[Figure 1] Figure 1 shows the partial genome structures of recombinant vaccinia viruses expressing one type of immune regulating gene.
[Figure 2-1] Figure 2-1 shows the observed cell images that indicate the cytopathic effect of vaccinia viruses carrying and expressing an immune regulating gene on A549 cells.
[Figure 2-2] Figure 2-2 shows the cell survival rates that indicate the cytopathic effect of vaccinia viruses carrying and expressing an immune regulating gene on A549 cells.
[Figure 3-1] Figure 3-1 shows the observed cell images that indicate the cytopathic effect of vaccinia viruses carrying and expressing an immune regulating gene on CT26 cells.
[Figure 3-2] Figure 3-2 shows the cell survival rates that indicate the cytopathic effect of vaccinia viruses carrying and expressing an immune regulating gene on CT26 cells.
[Figure 4] Figure 4 shows a protocol of a treatment experiment using a tumor bearing mouse model.
[Figure 5-1] Figure 5-1 shows the luminescence detection images that indicate viral growth in a tumor on the side where a vaccinia virus carrying and expressing an immune regulating gene was administered, and a distribution of viral growths.
[Figure 5-2] Figure 5-2 shows the numerical values representing viral growth in a tumor on the side where a vaccinia virus carrying and expressing an immune regulating gene was administered.
[Figure 6-1] Figure 6-1 shows the luminescence detection images that indicate viral growth in a tumor on the side where a vaccinia virus carrying and expressing an immune regulating gene was not administered, and a distribution of viral growths.
[Figure 6-2] Figure 6-2 shows the numerical values representing viral growth in a tumor on the side where a vaccinia virus carrying and expressing an immune regulating gene was not administered.
[Figure 7-1] Figure 7-1 shows the tumor growth curves after administration of vaccinia viruses carrying and expressing one type of immune regulating gene. Figure 7-1A shows the tumor growth curves on the administration side, and Figure 7-1B shows the tumor growth curves on the non-administration side.
[Figure 7-2] Figure 7-2 shows the survival curves after administration of vaccinia viruses carrying and expressing one type of immune regulating gene.
[Figure 8-1] Figure 8-1 is the tumor growth curves after administration of vaccinia viruses carrying and expressing two types of immune regulating genes. Figure 8-1A shows the tumor growth curves on the administration side, and Figure 8-1B shows the tumor growth curves on the non-administration side.
[Figure 8-2] Figure 8-2 shows the survival curves after administration of vaccinia viruses carrying and expressing two types of immune regulating genes.
[Figure 9] Figure 9 shows the partial genome structures of a recombinant vaccinia virus expressing one type of immune regulating gene.
[Figure 10] Figure 10 shows the tumor growth curves after administration of vaccinia viruses carrying and expressing one type of immune regulating gene. Figure 10A shows the tumor growth curves on the administration side, and Figure 10B shows the tumor growth curves on the non-administration side.
[Figure 11] Figure 11 shows the tumor growth curves after administration of vaccinia viruses carrying and expressing two types of immune regulating gene. Figure 11A shows the tumor growth curves on the administration side, and Figure 11B shows the tumor growth curves on the non-administration side.
[Figure 12] Figure 12 shows the partial genome structures of recombinant vaccinia viruses expressing two types of immune regulating genes.
[Figure 13] Figure 13 shows the expression levels of immune regulating genes in A549 cells infected with a vaccinia virus carrying and expressing the immune regulating genes. Figure 13A shows the expression level of mIL12, and Figure 13B shows the expression level of mCCL21.
[Figure 14] Figure 14 shows the tumor growth curves after administration of recombinant vaccinia viruses expressing two types of immune regulating genes. Figure 14A shows the tumor growth curves on the administration side, and Figure 14B shows the tumor growth curves on the non-administration side.
[Figure 15] Figure 15 shows the tumor growth curves after administration of a recombinant vaccinia virus expressing two types of immune regulating genes and having a cell fusion ability and a recombinant vaccinia virus expressing two types of immune regulating genes and not having a cell fusion ability. Figure 15A shows the tumor growth curves on the administration side, and Figure 15B shows the tumor growth curves on the non-administration side.
[Figure 16] Figure 16 is the survival curves after administration of a recombinant vaccinia virus expressing two types of immune regulating genes and having a cell fusion ability and a recombinant vaccinia virus expressing two types of immune regulating genes and not having a cell fusion ability.
[Figure 17] Figure 17 shows the tumor growth curve after administration of recombinant vaccinia viruses comprising a combination of three types of immune regulating genes. Figure 17A shows the tumor growth curve on the administration side, and Figure 17B shows the tumor growth curve on the non-administration side.
[Figure 18] Figure 18 shows the survival curve after administration of recombinant vaccinia viruses comprising a combination of three types of immune regulating genes.

### Description of Embodiments

The present invention is described in detail below.

The present invention provides a method for preparing a vaccinia virus expressing an exogenous therapeutic gene and the obtained vaccinia virus expressing a specific exogenous gene. The vaccinia virus expressing an exogenous therapeutic gene can be suitably used for cancer treatment.

Vaccinia virus strains used for manufacturing the vaccinia virus of the present invention are not limited, and examples thereof include the Lister strain and the LC16 strain, the LC16mO strain, and the LC16m8 strain, which were established from the Lister strain (for example, So Hashizume, Clinical Virology, Vol.3, No.3 (1975), p.269), the New York City Board of Health (NYBH) strain, the Wyeth strain, the Copenhagen strain, the Western Reserve (WR) strain, the Modified Vaccinia Ankara (MVA) strain, the EM63 strain, the Ikeda strain, the Dalian strain, and the Tian Tan strain. The LC16mO strain is a strain constructed from the Lister strain via the LC16 strain. The LC16m8 strain is an attenuated strain constructed further from the LC16mO strain in which the B5R gene, a gene encoding a viral membrane protein, has a frame shift mutation, and this protein is thereby prevented from being expressed and functioning (Protein, Nucleic Acid and Enzyme, Vol.48, No.12 (2003), pp.1693-1700).

In view of safety established for administration to humans, it is preferable that the vaccinia viruses used in the present invention have been attenuated and do not have pathogenicity. Examples of such attenuated strains include strains in which the B5R gene has been partially or completely deleted. The B5R gene encodes a protein existing in the envelope of a vaccinia virus, and the B5R gene product is involved in viral infection and growth. The B5R gene product exists in the surface of an infected cell and the envelope of the virus, has a role of increasing the infection efficiency when the virus infects and propagates in adjacent cells or other sites in the body of the host, and is also associated with the plaque size and the host range of the virus. When a virus from which the B5R gene has been deleted infects animal cells, the plaque size, as well as the pock size, is reduced. Further, the ability to grow in the skin is reduced, and the skin pathogenicity is therefore reduced. When the B5R gene has been partially or completely deleted in a vaccinia virus, the gene product of the B5R gene does not function normally, leading to a reduced skin growth. When the virus is administered to humans, it does not cause adverse reactions. Examples of attenuated strains from which the B5R gene has been deleted include the m8Δ strain (also referred to as LC16m8Δ strain), which was established by completely deleting the B5R gene from the above-mentioned LC16m8 strain. Further, the mOΔ strain (also referred to as LCmOΔ strain), which was established by completely deleting the B5R gene from the LC16mO strain, can also be used. These attenuated vaccinia virus strains from which the B5R gene has been partially or completely deleted are described in International Publication No. WO 2005/054451 and can be obtained according to the description therein. Whether the B5R gene has been partially or completely deleted, and the function of the B5R protein has been lost in a vaccinia virus can be assessed by using, for example, the sizes of plaques and pocks formed when the virus has infected RK13 cells, viral growth in Vero cells, and skin pathogenicity in rabbits as indicators. Further, the gene sequence of the vaccinia virus can be determined.

A vaccinia virus carrying the B5R gene expresses the B5R gene in a cancer cell and damages the cancer cell by the effect of the B5R protein. It is therefore recommended that the vaccinia virus used in the present invention expresses the B5R gene completely. When a vaccinia virus for which safety has been established because the virus does not carry the B5R gene and has been attenuated as described above is used, the complete B5R gene is introduced anew into the vaccinia virus from which the B5R gene has been deleted. A vaccinia virus from which the B5R gene has been partially or completely deleted can be used after inserting the B5R gene into the vaccinia virus genome. The B5R gene may be inserted into a vaccinia virus by any method, but can be done by, for example, a known homologous recombination technique. In this case, the position at which the B5R gene is inserted may be between the B4R gene and B6R gene, where the B5R gene originally exists, or at an arbitrary site in the vaccinia virus genome. Further, the B5R gene may be constructed as a DNA construct beforehand, and the DNA construct may be introduced into a vaccinia virus.

Examples of the vaccinia virus carrying an exogenous therapeutic gene of the present invention include vaccinia viruses described in International Publication No. WO 2011/125469, International Publication No. WO 2015/076422, and International Publication No. WO 2017/014296.

International Publication No. WO 2011/125469 describes a vaccinia virus in which a marker gene was inserted into endogenous genes, such as the TK gene and the HA gene, resulting in loss of the functions of TK and HA.

International Publication No. WO 2015/076422 describes a vaccinia virus in which an exogenous gene was inserted into the vaccinia virus growth factor (VGF) gene and the O1L gene, resulting in loss of the functions of the vaccinia virus growth factor (VGF) and O1L of the vaccinia virus. This vaccinia virus is referred to as a mitogen-activated protein kinase (MAPK)-dependent recombinant vaccinia virus (MDRVV).

International Publication No. WO 2017/014296 describes a vaccinia virus in which the UCA1 gene was introduced so as to express the gene.

Further, as the vaccinia virus of the present invention, a vaccinia virus which has been mutated so as to have a cell fusion ability can also be used. The term "cell fusion ability" used herein refers to an ability to cause fusion of infected cells when a vaccinia virus has infected the cells. The vaccinia virus that has been mutated so as to have a cell fusion ability is deficient in the function of a gene inherently carried by a vaccinia virus to regulate the cell fusion ability, or a gene which promotes cell fusion has been inserted into and expressed therein. The vaccinia virus that has been mutated so as to have a cell fusion ability is referred to as a fusogenic oncolytic vaccinia virus (FUVAC).

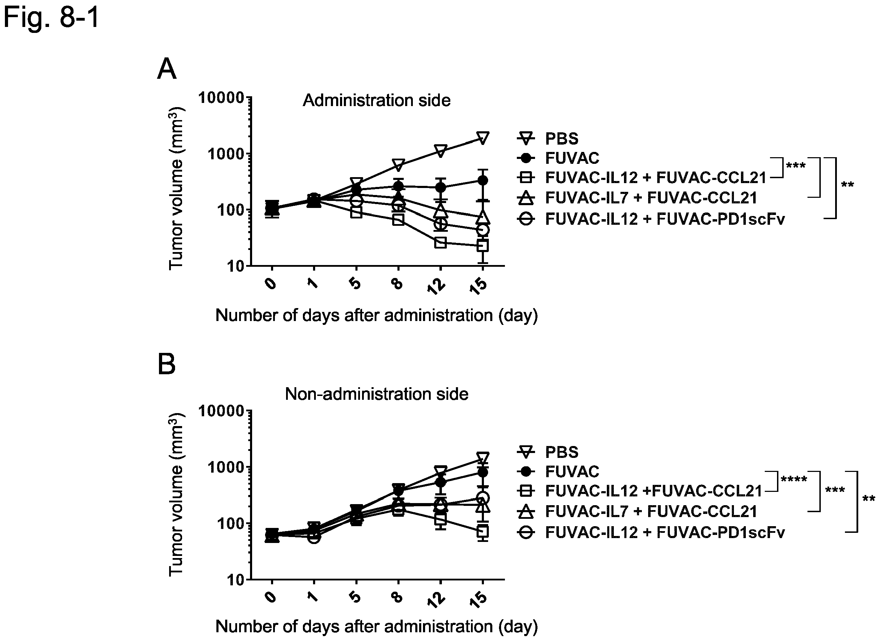

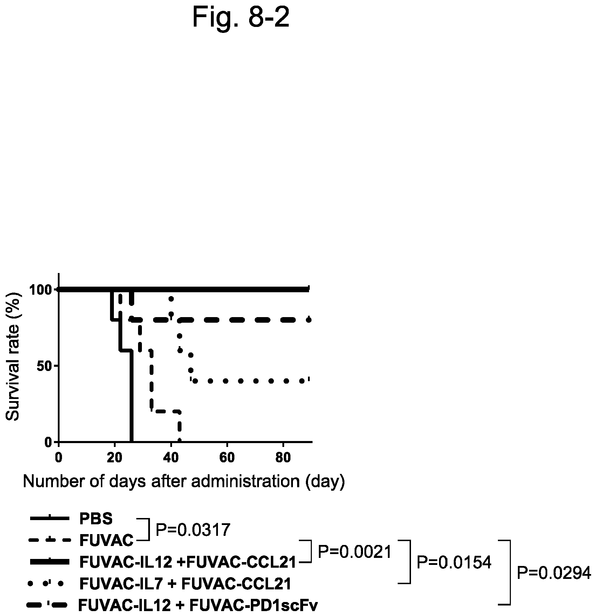

Examples of the gene which is involved in cell fusion and inherently carried by a vaccinia virus to regulate the cell fusion ability include the K2L gene and the HA (A56R) gene. The vaccinia virus of the present invention is deficient in the functions of the K2L gene or the HA gene or the K2L gene and the HA gene, and the phenotype thereof has therefore been changed to have the cell fusion ability. As shown in Figure 8 on p.5159 of Wagenaar et al., Journal of Virology, Vol.82, No.11 (June 2008), pp.5153-5160, a complex of the HA (A56R) protein and the K2L protein is immobilized on the cell membrane via the HA transmembrane region in a cell infected with a vaccinia virus. The entry/fusion complex (EFC) composed of a plurality of viral proteins (A21L, A28L, G3L, H2R, J5L, L5R) is immobilized on the membrane of a mature virus in coordination with viral proteins G9R and A16L. It is considered that fusion of cells infected with the virus is inhibited by these G9R and A16L, which act on HA and K2L on the cell membrane. Consequently, the impaired function of HA and K2L leads to loss of an inhibiting function and thereby enables induction of cell fusion. Therefore, examples of the gene involved in cell fusion and inherently carried by a vaccinia virus to regulate the cell fusion ability include the A16L, A21L, A25L, A26L, A28L, G3L, G9R, H2R, J5L, and L5R genes in addition to K2L and HA, which encode viral proteins. For example, fusion is induced by mutating H to Y at position 44 in the G9R gene even if the molecule which regulates fusion is normal. Therefore, cell fusion can be induced or enhanced by causing loss of one or a plurality of these functions or introducing a mutation. Examples of a combination thereof include deleting the K2L gene and mutating H to Y at position 44 in the G9R gene.

Several viral proteins having the fusion ability are known, and viruses can be mutated so as to have a cell fusion ability by inserting the genes thereof into viruses including different oncolytic viruses and expressing them. Examples of such genes include the genes encoding the H (hemagglutinin) protein and the F (fusion) protein derived from measles virus. Further, as shown in US Patent No. 7,635,752 specification, fusion can also be caused in specific cells by improving the H gene, and it has been demonstrated that the invention can be applied in cancer treatment by allowing the improved gene to be expressed in adenovirus or vesicular stomatitis virus (VSV) (Nakamura et al., Nature Biotechnology, Vol.22, No.3 (March 2004), pp.331-336). Further, the gene encoding the GaLV envelope derived from gibbon ape leukemia virus (GaLV) can also be mentioned, and it has also been demonstrated that the technique can be applied in cancer treatment by allowing the gene to be expressed in herpes virus, adenovirus, and lentivirus (Krabbe et al., Cancers, Vol.10 (2018), p.216; doi: 10.3390/cancers10070216).

Further, a VSV which expresses the FAST protein derived from reovirus, an adenovirus which expresses the HIV envelope derived from HIV, a VSV which expresses the F protein derived from Newcastle disease virus (NDV), and an adenovirus which expresses the F protein derived from SV5 have been reported (Krabbe et al., Cancers, Vol.10 (2018), p.216; doi: 10.3390/cancers10070216).

That is, examples of the genes promoting cell fusion also include the gene encoding the FAST protein derived from reovirus, the gene encoding the HIV envelope derived from HIV, the gene encoding the F protein derived from NDV, and the gene encoding the F protein derived from SV5.

The K2L gene is known as a serine protease inhibitor, but there are many unclear points in the function thereof. The HA gene is a glycoprotein induced on the surface of an infected cell and is known as a hemagglutinin. The nucleotide sequence of the wild-type K2L gene is shown in SEQ ID NO: 15, and the nucleotide sequence of the wild-type HA gene is shown in SEQ ID NO: 16.

Loss of the function of the K2L gene or the HA gene in a vaccinia virus means that the K2L gene or the HA gene is not expressed, or, if expressed, the expressed K2L or HA protein does not have a normal function thereof. To cause loss of the function of the K2L gene or the HA gene in a vaccinia virus, the whole or partial K2L gene or HA gene can be deleted. Further, the gene may also be mutated by substituting, deleting, or adding a nucleotide, so that the normal K2L protein or HA protein cannot be expressed. Further, an exogenous gene may also be inserted into the K2L gene or the HA gene. Of note, deficiency of K2L and HA caused cell fusion, but deficiency of other viral genes may also be utilized because it is causing cell fusion that is important for the anticancer effect in the present invention.

Loss of gene function can be caused by, for example, known methods such as genome editing, homologous recombination, RNA interference methods, antisense methods, gene insertion methods, artificial mutation methods, and PTGS methods using viral vectors. In the present invention, deficiency of a gene means that a normal gene product is not expressed because of deletion or mutation of the gene.

Homologous recombination is a phenomenon that two DNA molecules exchange the same nucleotide sequence with each other in a cell, which is a method often used for recombination of a virus having a very large genomic DNA, such as a vaccinia virus. First, a plasmid to which another DNA is ligated so that the sequence at the targeted K2L gene or HA gene site in a vaccinia virus is divided in the middle thereof is constructed. The constructed plasmid is referred to as a transfer vector. When the transfer vector is introduced into a cell infected with a vaccinia virus, recombination occurs between the viral DNA which has been made naked during the process of viral replication and the transfer vector having the same sequence portion, and the sandwiched DNA is incorporated into the target gene of the viral genome, resulting loss of the function of the gene. Examples of the cell used herein include cells which a vaccinia virus is capable of infecting, such as BSC-1 cells, HTK-143 cells, Hep2 cells, MDCK cells, Vero cells, HeLa cells, CV1 cells, COS cells, RK13 cells, BHK-21 cells, and primary rabbit kidney cells. Further, a vector can be introduced into a cell by a known method, such as a calcium phosphate method, a cationic ribosome method, and an electroporation method.

Genome editing is a method of modifying a target gene using a site-specific nuclease. Examples of the method of genome editing include, depending on the nuclease used, a zinc finger nuclease (ZFN) method (Urnov, Fyodor D et al., Nature, Vol.435 (2 June 2005), pp.642-651), a transcription activator-like effector nuclease (TALEN) method (Mahfouz, Magdy M et al., PNAS 108(6) (February 8, 2011), pp.2623-2628), and methods involving the Clustered Regularly Interspaced Short Palindromic Repeats (CRISPR)/Crispr Associated protein (Cas) system, such as CRISPR/Cas9 (Jinek M et al., Science, Vol.337 (17 August 2012), pp.816-821) and CRISPR/Cas3. These methods also include methods in which a nuclease is modified, such as a method using nickase-modified Cas. Among them, the method involving the CRISPR/Cas9 system is preferred. In the CRISPR/Cas9 system, an arbitrary sequence is cleaved with a guide RNA (crRNA or tracrRNA) comprising a sequence complementary to the target sequence of a gene whose function is to be lost by cleavage and a nuclease Cas9. After cleavage of the genome, repair occurs by non-homologous end joining (NHEJ), deficiency of a nucleotide or the like is induced, and the gene can be knocked out. Alternatively, after cleavage of the genome, homology-directed repair (HDR) occurs, and mutation can be induced in the target gene by homologous recombination. When the GBSS gene and/or the SBE gene are destroyed by genome editing, a target sequence in the gene is selected, and the sequence of a guide RNA comprising a sequence complementary to this sequence can be designed. The length of the guide RNA is preferably 20 or more nucleotides. When genome editing is performed using the CRISPR/Cas9 system, the Cas9 protein and the guide RNA can be coexpressed. For example, a vector expressing both thereof can be introduced. Genome editing involving CRISPR/Cas9 can be performed using a commercially available CRISPR/Cas9 tool.

When a vaccinia virus is deficient in the K2L gene or the HA gene, and the function of K2L or HA is lost, the oncolytic ability is improved by the enhanced growing and propagating ability of the virus, further inducing death of infected cancer cells. Here, the cell death includes apoptosis and necrosis. Further, cell fusion between infected cancer cells is caused. The immunogenic cell death (ICD)-inducing ability is improved by causing cell fusion. As a result, CD8 T cells are profusely infiltrated into cancer cells and attack the cancer cells. Further, the systemic anticancer immune activity is improved. Further, reduction of immunosuppressive cells, such as Treg, TAM, and MDSC, is also observed.

As a result, the deficiency of the K2L gene or the HA gene and the loss of the function of K2L or HA in a vaccinia virus improve the anticancer effect of the vaccinia virus.

As described above, by causing cell fusion in a cell infected with a vaccinia virus, the anticancer effect is improved, and the anticancer effect and the ICD inducing ability are improved regardless of the presence or absence of tumor specificity. However, if the vaccinia virus has tumor specificity, the anticancer effect is further improved synergistically. Therefore, it is preferable that the vaccinia virus used in the present invention is an oncolytic virus that has a tumor cell-specific cytolytic property and is capable of infecting cancer cells to kill the cancer cells. As a method thereof, a gene is modified to cause loss of the function of a specific protein or suppression of expression of a specific gene or protein.

Examples of such a gene include the hemagglutinin (HA) gene; the thymidine kinase (TK) gene; the F fragment; the F3 gene; the vaccinia virus growth factor (VGF) gene (US Patent Application Publication No. 2003/0031681 specification); O1L; hemorrhage region or A-type inclusion region (US Patent No. 6,596,279 specification); the *Hind*III F, F13L, or *Hind*III M region (US Patent No. 6,548,068 specification); the A33R, A34R, or A36R gene (Katz et al., J Virology, Vol.77 (2003), pp.12266-12275); the Sa1F7L gene (Moore et al., EMBO J, Vol.11 (1992), pp.1973-1980); the N1L gene (Kotwal et al., Virology, Vol.171 (1989), pp.579-58); the M1 gene (Child et al., Virology, Vol.174 (1990), pp.625-629); the HR, *Hind*III-MK, *Hind*III-MKF, *Hind*III-CNM*,* RR, or *Bam*F region (Lee et al., J Virol, Vol.66 (1992), pp.2617-2630); and the C21L gene (Isaacs et al., Proc Natl Acad Sci USA, Vol.89 (1992), pp.628-632). Among these genes, the VGF gene, O1L gene, TK gene, HA gene, and F fragment are preferred.

Further, a plurality of gene modifications may be performed in combination. Examples of a plurality of gene modifications include the following modifications:
- Loss of functions of TK and HA and F14.5L (Cancer Research, Vol.67 (2007), pp.10038-10046) and loss of functions of TK and B18R (PLoS Medicine, Vol.4 (2007), p.e353)
- Loss of functions of TK and ribonucleotide reductase (PLoS Pathogens, Vol.6 (2010), p.e1000984)
- Loss of functions of SPI-1 and SPI-2 (Cancer Research, Vol.65 (2005), pp.9991-9998)
- Loss of functions of SPI-1, SPI-2, and TK (Gene Therapy, Vol.14 (2007), pp.638-647)
- Introduction of mutation into the E3L and K3L regions (International Publication No. WO 2005/007824)

A plurality of these genes may be deleted. For example, two genes, the VGF gene and the O1L gene, may be deleted. A vaccinia virus in which functions of two genes, the VGF gene and the O1L gene, have been lost is described in International Publication No. WO 2015/076422.

For example, when the function of the TK gene is lost, the growing ability of a vaccinia virus in normal cells is reduced. However, the growing ability is not reduced in cancer cells because cancer cells have abundant enzymes that make up for the function of this gene therein. Reduction in the growing ability in normal cells means that pathogenicity against normal cells is reduced. In other words, safety is improved when the vaccinia virus is used in a living body. Further, when a vaccinia virus deficient in the functions of the two genes, the VGF gene and the O1L gene, infects normal cells, cell growth is not promoted because ERK is not activated in normal cells. As a result, the growth of the vaccinia virus is markedly reduced. However, since the Ras/Raf/MEK/ERK metabolic pathway is abnormally activated in cancer cells and makes up for the function of VGF and O1L to activate ERK in the vaccinia virus, the vaccinia virus can grow. As a result, the vaccinia virus grows specifically in cancer cells and destroys and damages cancer cells.

By using an oncolytic vaccinia virus, the ability of killing cancer cells is improved synergistically along with the cell fusion ability, which is increased by deleting the K2L gene or the HA gene.

These genes can be deleted by the above-described genome editing, homologous recombination, RNA interference methods, antisense methods, gene insertion methods, artificial mutation methods, PTGS methods using a viral vector, and the like.

A vaccinia virus having an oncolytic property is referred to as an oncolytic vaccinia virus.

The vaccinia virus of the present invention is an oncolytic vaccinia virus comprising an exogenous therapeutic gene.

Examples of the therapeutic gene include genes encoding physiologically active substances such as cytokines and chemokines, including interleukin-1 (IL-1), IL-2, IL-3, IL-4, IL-5, IL-6, IL-7, IL-8, IL-9, IL-10, IL-11, IL-12, IL-13, IL-14, IL-15, IL-17, IL-18, IL-21, IL-24, chemokine 2 (CCL2), CCL5, CCL19, CCL21, CXCL9, CXCL10, CXCL11, CD40L, CD70, CD80, CD137L, OX40L, GITRL, LIGHT, α-interferon, β-interferon, γ-interferon, GM-CSF, G-CSF, M-CSF, MIP1a, FLT3L, HPGD, TRIF, DAI, and tumor necrosis factor; and genes encoding immune checkpoint inhibitors such as antibodies having an inhibitory effect on CTLA4, PD1, and PD-L1. These genes encode proteins which are capable of regulating immunity, and are referred to as immune regulating genes. They are also referred to as immune activation genes because they have an immune activating effect.

A gene encoding an antibody may be a gene encoding a full-length antibody, but may be a gene encoding a functional fragment of the antibody. The functional fragment of an antibody is a fragment comprising an antigen-binding site in the variable region of the antibody, which is a part of a full-length antibody, and a fragment having an antigen-binding activity. Examples of the functional fragment of an antibody include Fab, Fab', F(ab')₂, and Fv fragments, diabody, and a single-chain antibody molecule (scFv), and these antibody fragment polymers are also included in the functional fragment of an antibody of the present invention. Fab is a fragment that can be obtained by treating an antibody with a proteolytic enzyme papain and an antibody fragment having a molecular weight of approximately 50,000 which is obtained by binding approximately a half of the H chain on the amino-terminus side and the whole L chain by a disulfide bond and has an activity of biding to an antigen. F(ab')₂ is an antibody fragment having a molecular weight of approximately 100,000 to which Fab is bound via a disulfide bond in the hinge region, among the fragments obtained by treating IgG with a proteolytic enzyme pepsin. Fab' is an antibody fragment having a molecular weight of approximately 50,000 in which the disulfide bond in the hinge region in the above-described F(ab')₂ has been cleaved. A single-chain antibody molecule (scFv), which is one of Fv fragments, is an antibody fragment in which one heavy-chain variable region (VH) and one light-chain variable region (VL) are linked via a peptide linker. A diabody is an antibody fragment obtained by dimerizing scFv and has a divalent antigen binding activity.

Further, examples of the therapeutic gene include tumor suppressor genes, such as p53 and Rb, and neovascularization inhibitor genes, such as angiostatin, thrombospondin, endostatin, METH-1, and METH-2.

When the vaccinia virus of the present invention is used for cancer treatment, a therapeutic gene against cancer can exhibit a cancer treatment effect along with the oncolytic property of the vaccinia virus.

Further, by introducing a DNA encoding an antigen such as a virus, bacterium, protozoan, and cancer as an exogenous gene (exogenous DNA), vaccinia virus vectors into which the exogenous gene has been introduced can be used as vaccines against various viruses, bacteria, protozoa, and cancers. For example, genes encoding antigens defensing against infection with (neutralizing antigens against) human immunodeficiency virus, hepatitis virus, herpes virus, mycobacteria, malaria protozoa, severe acute respiratory syndrome (SARS) virus, and the like; or genes encoding cancer antigens such as proteins such as WT1, MART-1, NY-ESO-1, MAGE-A1, MAGE-A3, MAGE-A4, Glypican-3, KIF20A, survivin, AFP-1, gp100, MUC1, PAP-10, PAP-5, TRP2-1, SART-1, VEGFR1, VEGFR2, NEIL3, MPHOSPH1, DEPDC1, FOXM1, CDH3, TTK, TOMM34, URLC10, KOC1, UBE2T, TOPK, ECT2, MESOTHELIN, NKG2D, P1A, 5T4, B7-H6, BCMA, CD123, CD133, CD138, CD171, CD19, CD20, CD22, CD23, CD30, CD33, CD38, CD44, CEA, c-MET, CS1, EGFR, EGFRvIII, EphA2, ErbB2, FAP, FR-α, HER2, IL13Ra2, MUC1, MUC16, NKG2D, PSCA, PSMA, ROR1, TARP, DLL3, PRSS21, Claudin18.2, Claudin18, CAIX, L1-CAM, FAP-α, CTAG1B, and FR-α and glycolipids such as GD2 and GM2 can be introduced.

These exogenous genes can be introduced by using, for example, a homologous recombination technique. Homologous recombination can be performed by the above-described methods. For example, a plasmid in which an exogenous gene to be introduced is linked to a DNA sequence at a target site for introduction (transfer vector) can be prepared and introduced into a cell infected with a vaccinia virus. Recombination occurs between a viral DNA which has been made naked during viral replication and the transfer vector having the same sequence portion, and the sandwiched exogenous gene is incorporated into the viral genome. Examples of cells that can be used here include cells that a vaccinia virus is capable of infecting, such as CV1 cells, RK13 cells, BSC-1 cells, HTK-143 cells, Hep2 cells, MDCK cells, Vero cells, HeLa cells, COS cells, BHK-21 cells, and primary rabbit kidney cells. Further, the vector can be introduced into cells by a known method, such as a calcium phosphate method, a cationic ribosome method, and an electroporation method.

The region into which the exogenous gene is introduced is preferably in a gene that is not essential to the life cycle of vaccinia virus. For example, the exogenous gene can be introduced into the vaccinia virus growth factor (VGF) gene or the O1L gene.

Further, when an exogenous gene is introduced, it is recommended to ligate a suitable promoter to the upstream of the exogenous gene so that the promoter can function. The promoter is not limited, but the above-described PSFJ1-10, as well as PSFJ2-16, p7.5K promoter, p11K promoter, T7.10 promoter, CPX promoter, HF promoter, H6 promoter, T7 hybrid promoter, and the like can be used. An exogenous gene can be introduced into the vaccinia virus vector of the present invention by a known method for constructing a recombinant vaccinia virus vector, and can be performed according to, for example, the descriptions in Experimental Medicine: The Protocol Series (separate volume), Analytical Experiment Methods for Gene Introduction and Expression, Saito I, et al. ed., Yodosha Co., Ltd. (issued September 1, 1997), DNA Cloning 4: Mammal System (second edition), Glover DM et al. ed., translation supervisor Kato I, TaKaRa, EMBO Journal Vol.6 (1987), pp.3379-3384, and the like.

At least one exogenous gene is introduced. Further, two or more, that is, a plurality of exogenous genes may be introduced. For example, two, three, four, five, six, seven, eight, nine, ten, or more exogenous genes may be introduced. The introduced exogenous gene may be a full-length gene or a fragment at a site having a function. The expression "a site having a function" used herein refers to a site which can lead to the same function as that of a full-length protein when the gene is expressed as a protein. Further, the introduced exogenous gene is a DNA having a sequence identity of at least 85% or higher, preferably 90% or higher, more preferably 95% or higher, more preferably 97% or higher, more preferably 98% or higher, particularly preferably 99% or higher to the gene sequence of a wild-type exogenous gene when calculated using Basic Local Alignment Search Tool (BLAST) at the National Center for Biological Information or the like (for example, a default, that is an initially set parameter), and a gene encoding a protein that has an activity equivalent to that of a protein encoded by a wild-type gene also falls within the scope of the exogenous gene of the present invention. In the amino acid sequences of proteins encoded by these genes, at least one, preferably one or several (for example, one to ten, more preferably one to five, particularly preferably one or two) amino acids may be deleted from the amino acid sequence of the protein encoded by the wild-type gene; at least one, preferably one or several (for example, one to nine, more preferably one to five, particularly preferably one or two) amino acids are added to the amino acid sequence of the protein encoded by the wild-type gene, or at least one, preferably one or several (for example, one to nine, more preferably one to five, particularly preferably one or two) amino acids of the amino acid sequence shown in SEQ ID NO: 6 may be substituted with other amino acids. Proteins having such amino acid sequences are proteins having an activity equivalent to that of the protein encoded by the wild-type gene. Further, an amino acid sequence obtained by deleting, substituting, or adding one or several amino acids in the amino acid sequence of such a protein encoded by the wild-type gene has a sequence identity of at least 85% or higher, preferably 90% or higher, more preferably 95% or higher, more preferably 97% or higher, more preferably 98% or higher, particularly preferably 99% or higher to the amino acid sequence of the protein encoded by the wild-type gene when calculated using Basic Local Alignment Search Tool (BLAST) at the National Center for Biological Information or the like (for example, a default, that is, an initially set parameter), and a protein having such an amino acid sequence is a protein having an activity equivalent to that of the protein encoded by the wild-type gene.

Among the above-mentioned exogenous therapeutic genes, the genes encoding IL-12, CCL21, IL-7, PDlscFv (scFv of anti-PD-1 antibody), and CD40L (CD154) are preferred. Further, two types, three types, four types, or five types of these genes can be used in combination, and examples of the combination of two types include a combination of the genes encoding IL-12 and CCL21, a combination of the genes encoding IL-12 and IL-7, a combination of the genes encoding IL-12 and PD1scFv, a combination of the genes encoding IL-12 and CD40L (CD154), a combination of the genes encoding CCL21 and IL-7, a combination of the genes encoding CCL21 and PD1scFv, a combination of the genes encoding CCL21 and CD40L (CD154), a combination of the genes encoding IL-7 and PD1scFv, a combination of the genes encoding IL-7 and CD40L (CD154), and a combination of the genes encoding PDlscFv and CD40L (CD154). Examples of the combination of three types include a combination of the genes encoding IL-12, CCL21, and IL-7, a combination of the genes encoding IL-12, CCL21, and PD1scFv, a combination of the genes encoding IL-12, CCL21, and CD40L (CD154), a combination of the genes encoding IL-12, IL-7, and PD1scFv, a combination of the genes encoding IL-12, IL-7, and CD40L (CD154), a combination of the genes encoding IL-12, PD1scFv, and CD40L (CD154), a combination of the gene encoding CCL21, IL-7, and PD1scFv, a combination of the genes encoding CCL21, IL-7, and CD40L (CD154), a combination of the genes encoding CCL21, PD1scFv, and CD40L (CD154), and a combination of the genes encoding IL-7, PD1scFv, and CD40L (CD154). Examples of the combination of four types include a combination of the genes encoding IL-12, CCL21, IL-7, and PD1scFv, a combination of the genes encoding IL-12, CCL21, IL-7, and CD40L (CD154), a combination of the genes encoding IL-12, CCL21, PD1scFv, and CD40L (CD154), a combination of the genes encoding IL-12, IL-7, PD1scFv, and CD40L (CD154), and a combination of the genes encoding CCL21, IL-7, PD1scFv, and CD40L (CD154). Examples of the combination of five types include a combination of the genes encoding IL-12, CCL21, IL-7, PD1scFv, and CD40L (CD154).

Viruses into which an exogenous gene has been introduced are represented as MDRVV-IL12 (MDRVV into which the gene encoding IL-12 has been introduced), MDRVV-IL7/CCL21 (MDRVV into which two genes, the gene encoding IL-7 and the gene encoding CCL21, have been introduced), FUVAC-IL12 (FUVAC into which the gene encoding IL-12), and FUVAC-IL7/CCL21 (FUVAC into which two genes, the gene encoding IL-7 and the gene encoding CCL21, have been introduced).

A plurality of exogenous therapeutic genes may be introduced into one vaccinia virus to use the vaccinia virus for treatment. One exogenous gene may be introduced into one vaccinia virus, and a plurality of vaccinia viruses into each of which a different exogenous gene has been introduced may be used for treatment. For example, a vaccinia virus into which any one of the genes encoding IL-7, IL-12, CD40L (CD154), CCL21, and PDlscFv (scFv of anti-PD-1 antibody) has been introduced is prepared, a vaccinia virus into which the gene encoding IL-7 has been introduced, a vaccinia virus into which the gene encoding IL-12 has been introduced, a vaccinia virus into which the gene encoding CD40L (CD154) has been introduced, a vaccinia virus into which the gene encoding CCL21 has been introduced, and a vaccinia virus into which the gene encoding PDlscFv (scFv of anti-PD-1 antibody) has been introduced are prepared, and two, three, four, or five of these vaccinia viruses can be used in combination for treatment.

Further, a vaccinia virus into which one or a plurality of, for example, one or two exogenous genes have been introduced may be used in combination with a vaccinia virus into which one or a plurality of, for example, one or two different exogenous genes have been introduced. In this case as well, a plurality of vaccinia viruses can be used in combination. For example, a vaccinia virus into which the gene encoding any one of IL-7, IL-12, CD40L (CD154), CCL21, and PDlscFv (scFv of anti-PD-1 antibody) has been introduced may be used in combination with a vaccinia virus into which the genes encoding any two or more of IL-7, IL-12, CD40L (CD154), CCL21, and PDlscFv (scFv of anti-PD-1 antibody) which are different from the gene introduced into the above vaccinia virus have been introduced. Examples thereof include combinations of viruses comprising three types of exogenous genes, such as a combination of FUVAC-IL12/IL7 and FUVAC-CCL21, a combination of FUVAC-CCL21/IL7 and FUVAC-IL12, a combination of FUVAC-IL12/CCL21 and FUVAC-IL7, a combination of MDRVV-IL12/IL7 and MDRVV-CCL21, a combination of MDRVV-CCL21/IL7 and MDRVV-IL12, and a combination of MDRVV-IL12/CCL21 and MDRVV-IL7.

The present invention encompasses a composition comprising a plurality of vaccinia viruses into which different exogenous genes have been introduced and a therapeutic kit comprising a combination of separate compositions each comprising each of a plurality of vaccinia viruses into which different exogenous genes have been introduced.

The ability of killing cancer cells is improved synergistically by introducing the above-mentioned therapeutic genes into oncolytic vaccinia viruses and using the viruses.

Cancers treated by a cancer virotherapy using a vaccinia virus are not limited, and examples thereof include all cancer types, such as ovarian cancer, lung cancer, pancreatic cancer, skin cancer, stomach cancer, liver cancer, hepatocellular carcinoma, colon cancer, anal/rectal cancer, esophageal cancer, uterine cancer, breast cancer, bladder cancer, prostate cancer, testicular cancer, head/neck region cancer, brain/nerve tumor, thymic cancer, lymphoma/leukemia, bone cancer/osteosarcoma, leiomyoma, rhabdomyoma, and melanoma.

The pharmaceutical composition for cancer treatment comprising a vaccinia virus of the present invention contains a pharmaceutically effective amount of the vaccinia virus of the present invention as an active ingredient and may be in the form of an aseptic aqueous or nonaqueous solution, suspension, or emulsion. Further, the pharmaceutical composition may contain pharmaceutically acceptable diluents, auxiliaries, carriers, and the like, such as salts, buffers, and adjuvants. The pharmaceutical composition can be administered via various parenteral routes, such as, for example, a subcutaneous route, intravenous route, intracutaneous route, intramuscular route, intraperitoneal route, intranasal route, and percutaneous route. Further, the pharmaceutical composition can be locally administered into the cancer region. The effective dose can be determined suitably depending on the subject's age, sex, health, body weight, and the like. For example, the effective dose is, but not limited to, approximately 10² to 10¹⁰ plaque-forming unit (PFU) per administration for a human adult.

The present invention encompasses a method for cancer treatment comprising administering the above-described vaccinia virus to a cancer patient.

### Examples

The present invention is specifically described by the following examples, but the present invention is not limited to these examples.

### Example 1 Preparation of a recombinant vaccinia virus carrying and expressing an immune regulating gene

To insert a gene-expressing unit of different exogenous genes into the VGF gene and the O1L gene in a mitogen-activated protein kinase-dependent recombinant vaccinia virus in which neither VGF nor O1L functions (International Publication No. WO 2015/076422), the BFP gene region was amplified using the DNA of pTagBFP-N (FP172, Evrogen) as a template and two primers (SEQ ID NO: 1 and SEQ ID NO: 2). Each PCR product thereof was cleaved with restriction enzymes *Sfi*I and *Eco*RI and cloned at the same restriction enzyme sites in the pTK-SP-LG vector (International Publication No. WO 2015/076422), to construct pTNshuttle/TK-SP-BFP, in which BFP was ligated to the downstream of a synthesized vaccinia virus promoter (Hammond JM. et al., Journal of Virological Methods, Vol.66(1) (1997), pp.135-138). Subsequently, pTNshuttle/TK-SP-BFP was cleaved with restriction enzymes *Sph*I and *EcoR*I and subjected to blunt treatment, and an SP-BFP fragment thereof was cloned at a site at which a pUC19-VGF vector (International Publication No. WO 2015/076422) was cleaved with a restriction enzyme *Acc*I and subjected to blunt treatment, or a site at which a pUC19-O1L vector (International Publication No. WO 2015/076422) was cleaved with a restriction enzyme *Xba*I and subjected to blunt treatment to construct a shuttle vector pTNshuttle/VGF-ST-BFP to express BFP in the reverse direction to VGF or pTNshuttle/O1L-SP-BFP to express BFP in the reverse direction to O1L. Meanwhile, by the same method as described in International Publication No. WO 2015/076422, pUC19-O1L-p7.5-DsRed to express DsRed in the same direction as O1L was constructed in the downstream of p7.5K promoter (p7.5), not the synthesized vaccinia virus promoter (SP). CV1 cells cultured at 80% confluency in a 24-well plate were infected with MDRVV virus (International Publication No. WO 2015/076422) at MOI = 0.1 to 0.5 and adsorbed at room temperature for one hour. Subsequently, a transfer vector plasmid pTNshuttle/O1L-SP-BFP mixed with FuGENE HD (Roche) was added to cells to be taken up by the cells in accordance with the manual, and the cells were cultured at 37°C for two to three days. The cells were collected, frozen and thawed, and then sonicated, the virus was appropriately diluted and inoculated into the BSC1 cells that were almost confluent, followed by addition of an Eagle's MEM medium supplemented with 5% FBS which contained 0.5% methylcellulose, and the cells were cultured at 37°C for two to four days. The medium was removed, and plaques expressing BFP were scraped with the tip of a chip and suspended in the Opti-MEM medium (Invitrogen). This procedure was repeated at least three times using BSC1 cells to purify the plaques. The suspension of the plaques collected after purification of plaques was sonicated, and then the genomic DNA was extracted from 200 µL of the suspension using High Pure Viral Nucleic Acid Kit (Roche) in accordance with the manual and screened by PCR. To detect O1L, PCR was performed using two primers (SEQ ID NO: 3 and SEQ ID NO: 4), and the nucleotide sequence of a PCR product in a clone in which a PCR product having a predetermined size was detected was checked by direct sequencing. A viral clone VGF-LucGFP/O1L-BFP having no problem in the nucleotide sequence was selected and amplified in A549 cells, then the viral titer was measured in RK13 cells, and the clone was subjected to experiments. On the basis of this vaccinia virus (VGF-LucGFP/O1L-BFP) and the transfer vector plasmid DNA pUC19-O1L-p7.5-DsRed, a recombinant virus was collected by the same method as described above using the expression of DsRed as an indicator and designated as VGF-LucGFP/O 1L-DsRed.

During this process of constructing the recombinant vaccinia virus VGF-LucGFP/O1L-DsRed, a virus having a cell fusion ability that is not usually observed appeared in very high frequency. Accordingly, the medium was removed, and plaques having this fusion characteristic were scraped with the tip of a chip and suspended in the Opti-MEM medium (Invitrogen). This procedure was further repeated at least three times using BSC1 cells, and a viral clone FUVAC having a cell fusion ability was obtained by purification of the plaques. This clone was subjected to direct sequencing using a next-generation sequencer PacBio RSII (Pacific Bioscience) or by PCR, the obtained sequence information showed that there was a mutation in the K2L gene, and it was found that the 254th amino acid was mutated from tryptophan to a stop codon in this mutation, that is, a nonsense mutation occurred (Japanese Patent Application No. 2019-091609, PCT/JP2020/018976).

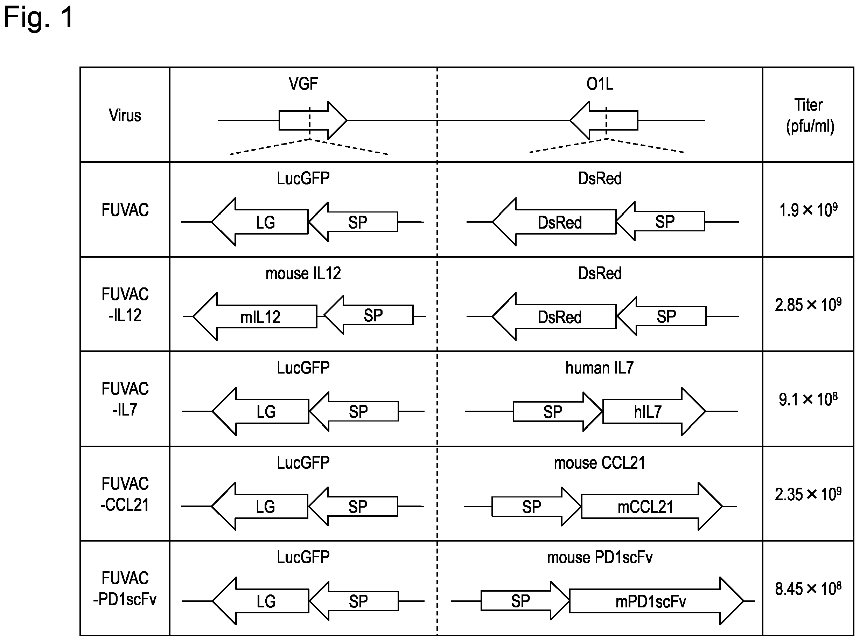

To collect a recombinant vaccinia virus having the viral genome shown in Figure 1, a gene obtained by adding restriction enzymes *Age*I and *Nhe*I to both ends of the mouse gene encoding IL-12, which is composed of an IL-12 p40 subunit (SEQ ID NO: 6) in the upstream of the IRES sequence derived from a encephalomyocarditis virus (SEQ ID NO: 5) and an IL-12 p35 subunit (SEQ ID NO: 7) in the downstream thereof, was synthesized and cleaved with *Age*I and *Nhe*I*,* and then cloned at the same restriction enzyme sites of the pTNshuttle/VGF-SP-BFP vector so as to substitute the gene with the BFP gene to construct pTNshuttle/VGF-SP-mIL12. Similarly, a gene obtained by adding restriction enzymes *Age*I and *Nhe*I to both ends of the gene encoding human IL-7 (SEQ ID NO: 8), mouse CCL21 (SEQ ID NO: 9), or mouse single-chain anti-PD1 antibody (scFv) (Oncoimmunology, Vol.5(10) (2016), p.e1220467) was synthesized and cleaved with *Age*I and *Nhe*I*,* and then cloned at the same restriction enzyme sites of the pTNshuttle/O1L-SP-BFP vector so as to substitute the gene with the BFP gene to construct pTNshuttle/O1L-SP-hIL7, pTNshuttle/O1L-SP-mCCL21, or pTNshuttle/O1L-SP-mPD1scFv. Subsequently, on the basis of the above-described vaccinia virus FUVAC and the transfer vector plasmid DNA pTNshuttle/VGF-SP-mIL12, a recombinant virus was collected by the same method as described above using loss of GFP expression as an indicator and designated as FUVAC-IL12. Similarly, on the basis of the vaccinia virus FUVAC and the transfer vector plasmid DNA pTNshuttle/O1L-SP-hIL7, pTNshuttle/O1L-SP-mCCL21, or pTNshuttle/O1L-SP-mPD1scFv, a recombinant virus was collected by the same method as described above using loss of DsRed expression as an indicator and designated as FUVAC-IL7, FUVAC-CCL21, or FUVAC-PD1scFv. PCR was performed using two primers shown in SEQ ID NO: 10 and SEQ ID NO: 11 to detect VGF and two primers shown in SEQ ID NO: 12 and SEQ ID NO: 13 to detect O1L, and the nucleotide sequence of a PCR product of a clone in which a PCR product having a predetermined size was detected was checked by direct sequencing. Each of recombinant viruses having no problem in the nucleotide sequence was cultured in A549 cells in a large amount and purified, then the viral titer was measured in RK13 cells, and the recombinant viruses were subjected to experiments.

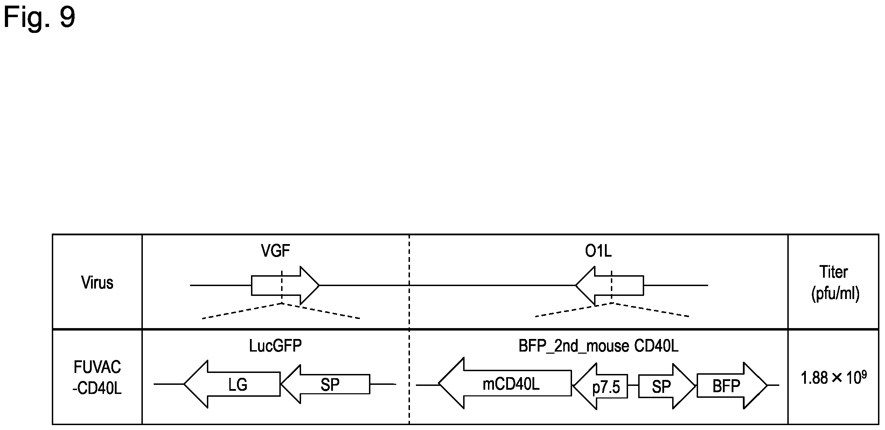

To collect a recombinant vaccinia virus having the viral genome shown in Figure 9, a sequence having restriction enzyme sites *Esp*EI and *Avr*II in the downstream of the p7.5K promoter (p7.5) was inserted into pTNshuttle/O1L-SP-BFP in the reverse direction to BFP expression to construct pTNshuttle/O1L-SP-BFP+p7.5. A gene obtained by adding restriction enzymes *Age*I and *Nhe*I to both ends of the gene encoding mouse CD40L (SEQ ID NO: 14) was synthesized and cleaved with *Age*I and *Nhe*I*,* and then cloned at restriction enzyme sites *Esp*EI and *Avr*II in the pTNshuttle/O1L-SP-BFP+p7.5 vector to construct pTNshuttle/O1L-SP-BFP+p7.5-mCD40L. Similarly, as described above, on the basis of the vaccinia virus FUVAC and the transfer vector plasmid DNA pTNshuttle/O1L-SP-BFP+p7.5-mCD40L, a recombinant virus was collected by the same method as described above using loss of DsRed expression and expression of BFP as indicators and designated as FUVAC-CD40L. Each of recombinant viruses having no problem in the nucleotide sequence was cultured in A549 cells in a large amount and purified by the above-described method, then the viral titer was measured in RK13 cells, and the recombinant viruses were subjected to experiments.

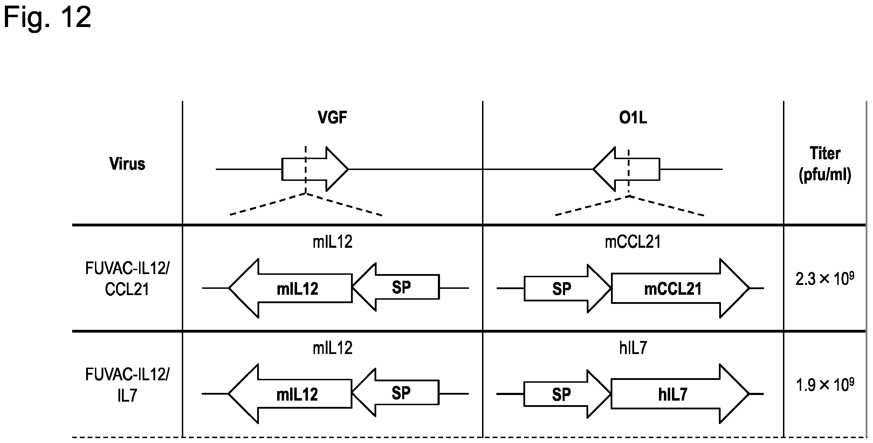

To collect a recombinant vaccinia virus having the viral genome shown in Figure 12, on the basis of the above-described vaccinia virus FUVAC and the transfer vector plasmid DNA pTNshuttle/VGF-SP-mIL12, the recombinant virus was collected by the same method as described above using loss of GFP expression as an indicator and designated as FUVAC-IL12. Subsequently, on the basis of the vaccinia virus FUVAC-IL12 and the transfer vector plasmid DNA pTNshuttle/O1L-SP-mCCL21 or pTNshuttle/O1L-SP-hIL7, a recombinant virus was collected by the same method as described above using loss of DsRed expression as an indicator and designated as FUVAC-IL12/CCL21 or FUVAC-IL12/IL7. Each of recombinant viruses having no problem in the nucleotide sequence was cultured in a large amount in A549 cells and purified by the above-described method, then the viral titer was measured in RK13 cells, and the recombinant viruses were subjected to experiments.

### Example 2 Analysis of characteristics of recombinant vaccinia viruses carrying and expressing an immune regulating gene

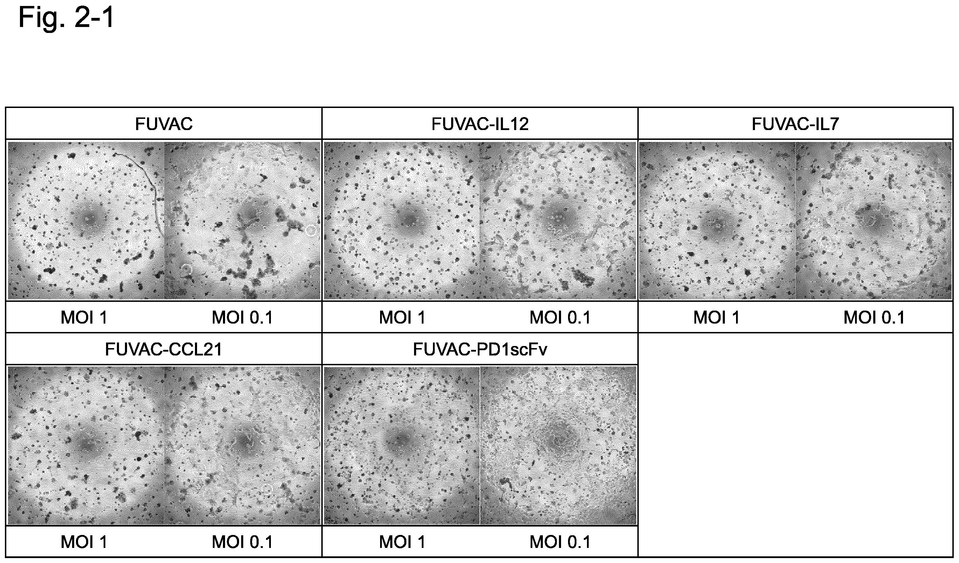

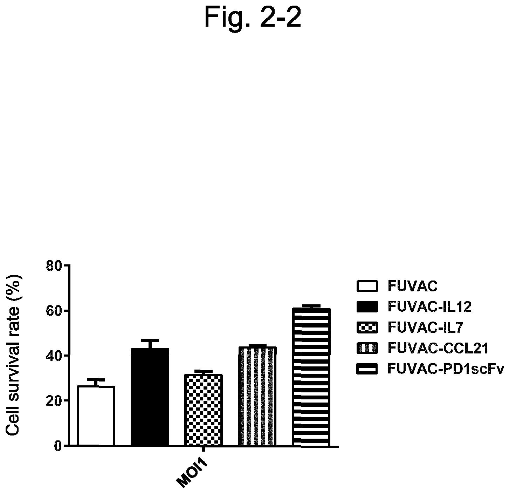

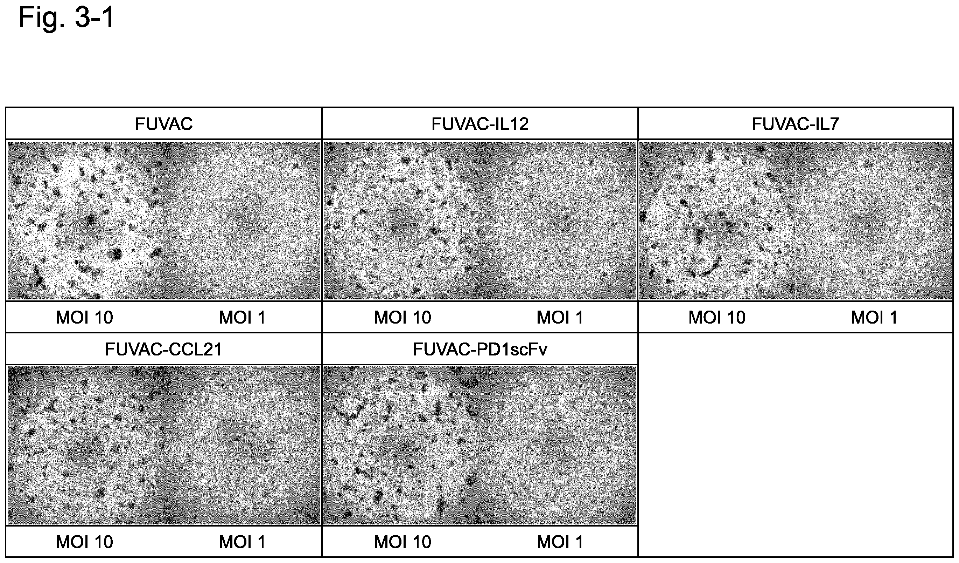

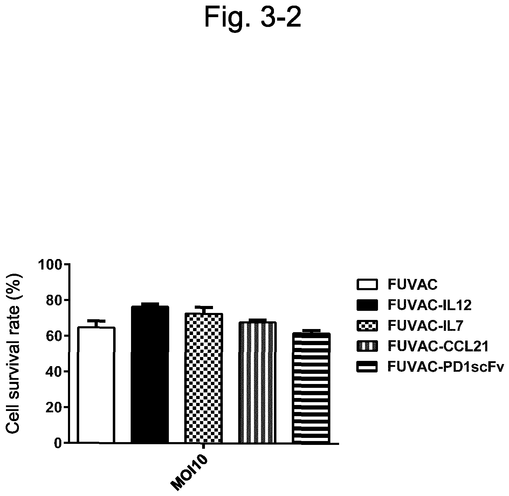

To compare recombinant vaccinia viruses carrying and expressing an immune regulating gene, each of the viruses was allowed to infect cancer cells. First, human lung cancer A549 cells or mouse colorectal cancer CT26 cells were seeded on a 96-well plate at 1.0 × 10⁴ cells/well and cultured for 24 hours, and then each of the viruses was allowed to infect A549 cells at MOI = 0.1 or 1 and CT26 cells at MOI = 1 or 10. At 72 hours after infection, the infection image was observed using BZ-X700 (Keyence Corporation). The results showed that, as compared with FUVAC not carrying or expressing an immune regulating gene, the FUVAC carrying and expressing an immune regulating gene infected, grew, and propagated in both A549 cells (Figure 2-1) and CT26 cells (Figure 3-1) regardless of the type of the carried and expressed gene, while fusing the cells dependently on the viral load. Further, at 72 hours after infection, the cell survival rate was measured using CellTiter 96 (registered trade name) Aqueous Non-radioactive Cell Proliferation Assay (Promega). The results showed that, as compared with FUVAC, FUVAC carrying and expressing an immune regulating gene exhibited a cytotoxic effect in both A549 cells (MOI = 1) and CT26 cells (MOI = 10) regardless of the type of the carried and expressed gene. Figure 2-2 and Figure 3-2 show the cell survival rate of each of virus-infected cells (n = 3) assuming the cell survival rate of mock cells, which were subjected to the same treatment without being infected with the virus, as 100%.

### Example 3 Treatment effect of recombinant vaccinia viruses carrying and expressing an immune regulating gene

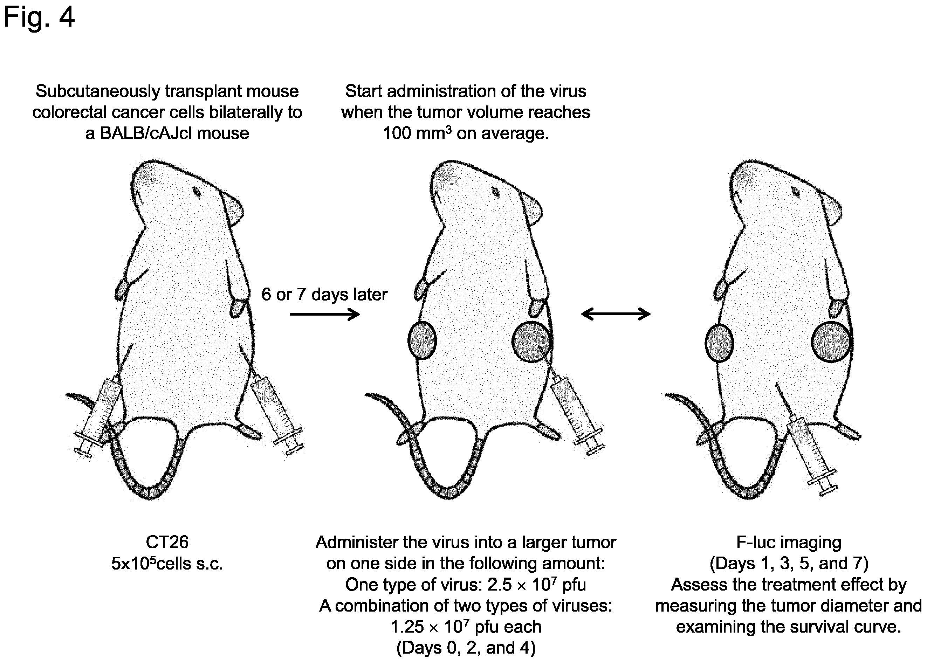

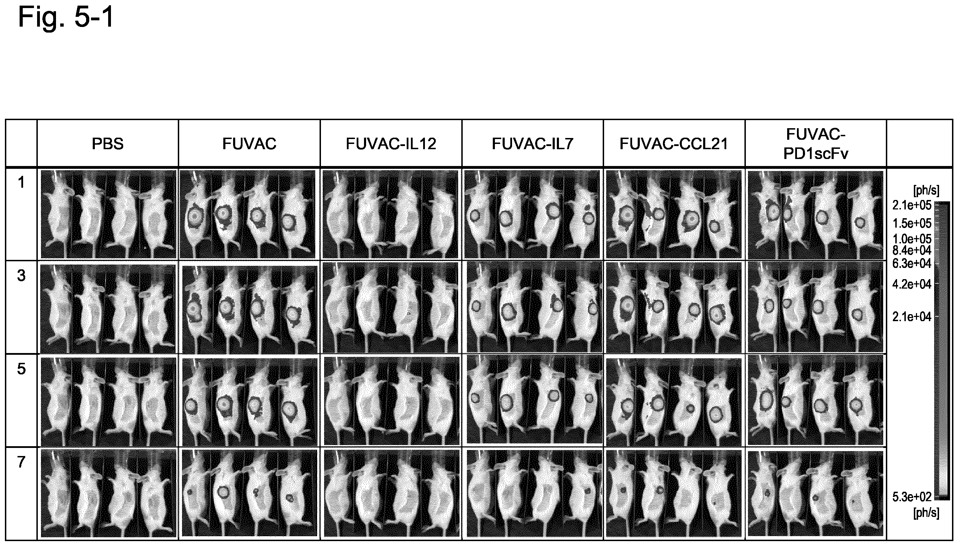

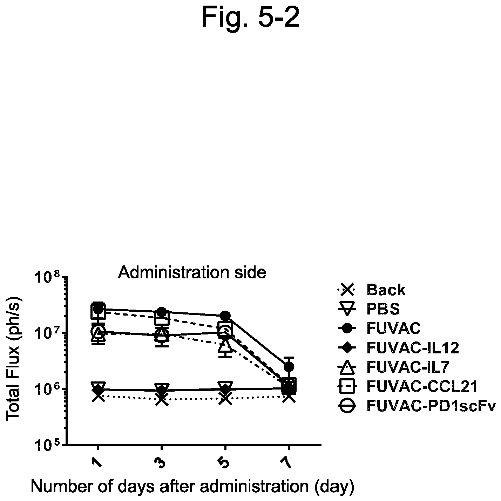

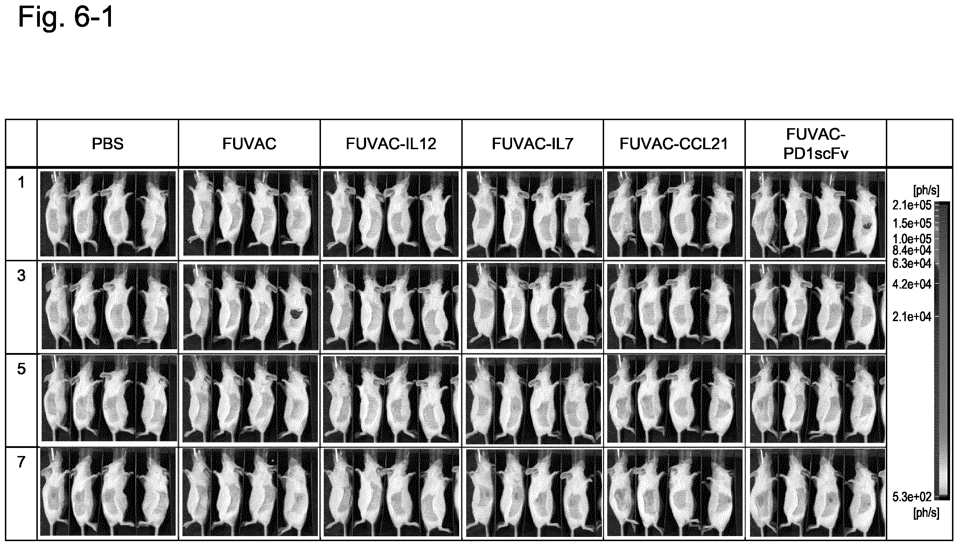

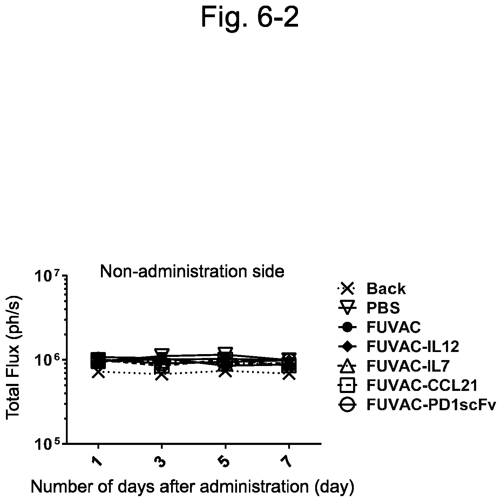

Viral growth and propagation in an organism and the treatment effect of viruses were investigated using an allogeneic transplantation model shown in Figure 4. CT26 cells were subcutaneously transplanted into both sides of the abdomen of a 6-week-old, female BALB/cAJcl mouse at 5.0 × 10⁵ cells/mouse. Tumors were allowed to grow for six to seven days until the tumor volume exceeded 100 mm³ on average. The tumor volume was calculated by an equation, minor diameter × major diameter × major diameter × 0.5. After tumor growth, PBS or each of viruses was administered directly into the tumor on one side at 2.5 × 10⁷ PFU three times every other day (Days 0, 2, and 4). Further, viral growth and propagation were noninvasively detected using an *in vivo* imaging system (Night SHADE LB985, Berthold Technologies) on Days 1, 3, 5, and 7 by a method of detecting the virus noninvasively by checking the presence or absence of luminescence by luciferase expressed in tumor cells infected with each of viruses carrying the luminescent enzyme after administering Vivo Glo Luciferin (Promega), which is a substrate thereof (luciferin). Figure 5-1 shows an image of detection of the virus FLuc after administration of the virus, and Figure 5-2 shows the result of quantification thereof. The virus FLuc on the virus administration side showed an equally high signal at Days 1, 3, and 5 after administration and disappeared uniformly at Day 7 after administration. Figure 6-1 shows an image of detection of virus FLuc after not administering the virus, and Figure 6-2 shows the result of quantification thereof. Viral signals were not observed on the virus non-administration side. From the above, as compared with FUVAC not carrying or expressing an immune regulating gene, there was no difference in viral growth in the tumor to which the FUVAC carrying and expressing an immune regulating gene was administered, regardless of the type of the carried and expressed gene, and it was found that the virus did not propagate into the tumor on the non-administration side.

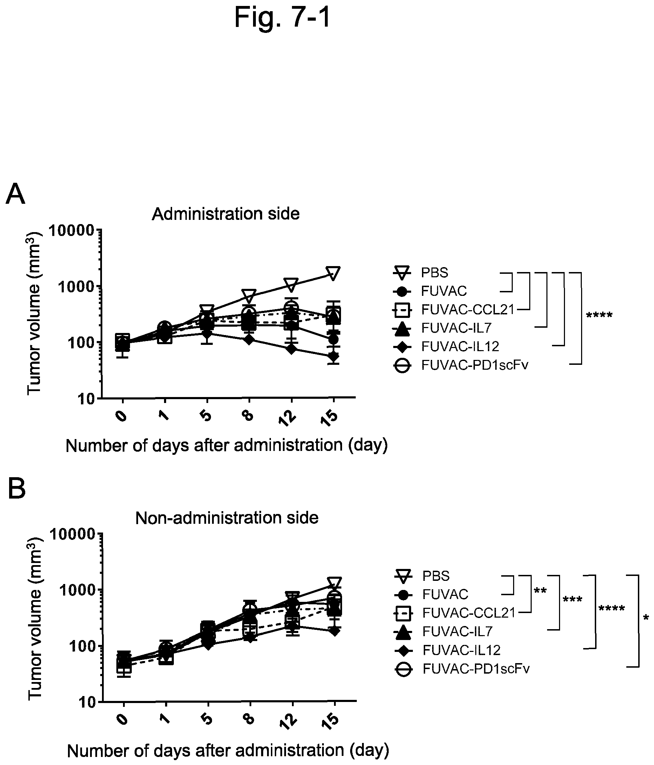

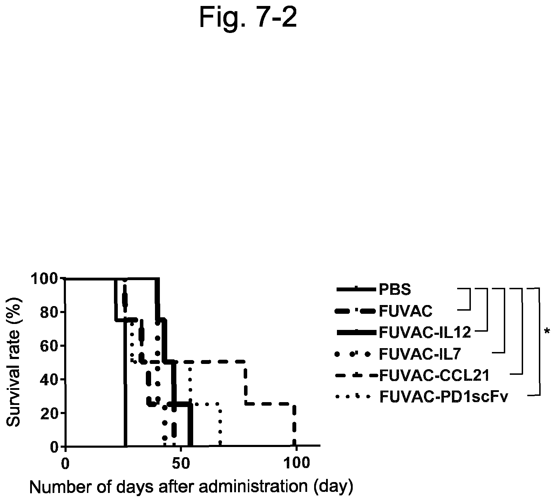

Subsequently, the treatment effect of the virus was investigated by measuring the tumor diameter and examining the survival curve. The results of a statistical analysis by two-way ANOVA demonstrated that, as compared with administration of PBS, the tumor diameter was significantly reduced on the virus administration side and the non-administration side after administration of FUVAC not carrying or expressing an immune regulating gene and each of the FUVACs carrying and expressing an immune regulating gene (Figure 7-1: ****, p < 0.0001; ***, p < 0.001; **, p < 0.01; *, p < 0.05). As compared with administration of FUVAC, there was no significant difference in the tumor diameter on the virus administration side and the virus non-administration side after administration of each of viruses carrying and expressing an immune regulating gene (Figure 7-1). A statistical analysis by the log-rank test showed that, as compared with mice given PBS, the survival time was significantly prolonged in mice given FUVAC not carrying or expressing an immune regulating gene and each of viruses carrying and expressing an immune regulating gene (*, p < 0.05). However, there was no significant difference in the survival time between mice given each of viruses carrying and expressing an immune regulating gene and mice given FUVAC (Figure 7-2).

### Example 4 Treatment effect by a combination of two types of recombinant vaccinia viruses carrying and expressing an immune regulating gene

Using the allogeneic transplantation model shown in Figure 4 as in Example 3, PBS or each of viruses was directly administered into a tumor on one side three times every other day (Days 0, 2, and 4). The treatment effect of the combinations of viruses was investigated by measuring the tumor diameter and examining the survival curve. The results of a statistical analysis by two-way ANOVA demonstrated that, as compared with administration of FUVAC not carrying or expressing an immune regulating gene, the tumor diameter was significantly reduced on the virus administration side and the virus non-administration side after administration of the combinations of FUVAC-IL12 and FUVAC-CCL21, FUVAC-IL12 and FUVAC-PD1, FUVAC-IL7 and FUVAC-CCL21, and FUVAC-IL7 and FUV AC-PD1 scFva (Figure 8-1: ****, p < 0.0001; ***, p < 0.001; **, p < 0.01). A statistical analysis by the log-rank test showed that, as compared with mice given FUVAC, the survival time was significantly prolonged in mice given the combinations of FUVAC-IL12 and FUVAC-CCL21, FUVAC-IL12 and FUVAC-PD1, FUVAC-IL7 and FUVAC-CCL21, and FUVAC-IL7 and FUVAC-PD1scFv (Figure 8-2). It is notable that complete remission of tumor was achieved on both sides in five of the five mice given FUVAC-IL12 and FUVAC-CCL21, four of the five mice given FUVAC-IL12 and FUVAC-PD1, and two of the five mice given FUVAC-IL7 and FUVAC-CCL21.

From the above results, as compared with monotherapy with a recombinant vaccinia virus carrying and expressing an immune regulating gene, the combination therapy exhibited a very high treatment effect that could not be expected not only on the virus administration side but also on the non-administration side, thus prolonging the survival time.

### Example 5 Treatment effect of combinations of two types of other recombinant vaccinia viruses carrying and expressing an immune regulating gene

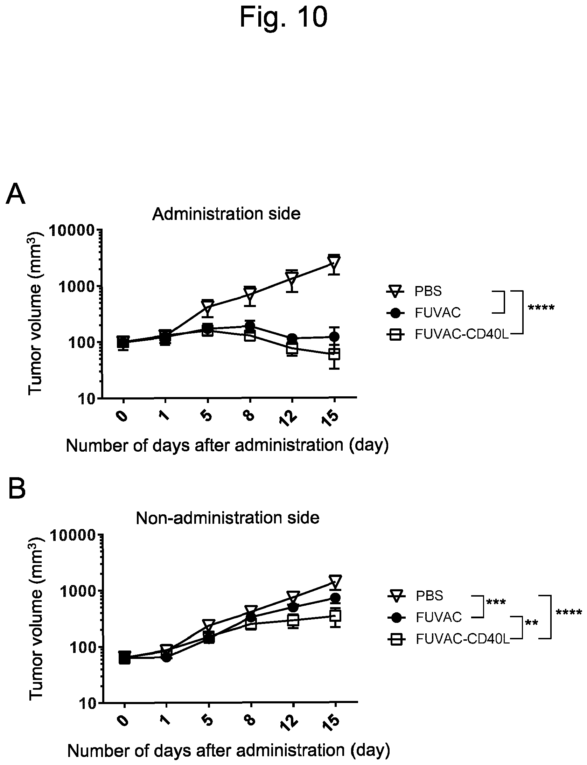

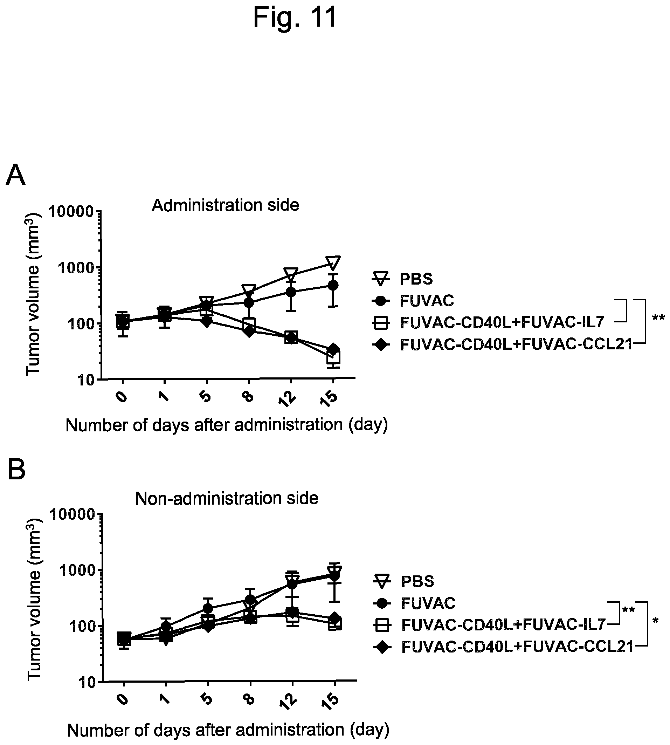

Using the allogeneic transplantation model shown in Figure 4 as in Example 3, the treatment effect of FUVAC-CD40L (Figure 9), which is a recombinant vaccinia virus carrying and expressing CD40L, was investigated by measuring the tumor diameter after PBS or each of viruses was directly administered into a tumor on one side three times every other day (Days 0, 2, and 4). The results of a statistical analysis by two-way ANOVA demonstrated that, as compared with administration of PBS, the tumor diameter was significantly reduced on the virus administration side and the non-administration side after administration of FUVAC-CD40L (Figure 10: ****, p < 0.0001; ***, p < 0.001). Further, two-way ANOVA demonstrated that there was no significant difference in the tumor diameter on the virus administration side after administration of FUVAC-CD40L as compared with FUVAC, but the tumor diameter was significantly reduced on the non-administration side (Figure 10: **, p < 0.01). Further, the treatment effect of a combination of FUVAC-CD40L and FUVAC-IL7 and FUVAC-CD40L and FUVAC-CCL21 was investigated. The results of a statistical analysis by two-way ANOVA demonstrated that, as compared with FUVAC, the tumor diameter was significantly reduced on the virus administration side and the non-administration side after administration of the combinations of FUVAC-CD40L and FUVAC-IL7 and FUVAC-CD40L and FUVAC-CCL21 (Figure 11: **, p < 0.01; *, p < 0.05).

### Example 6 Recombinant vaccinia viruses carrying and expressing two types of immune regulating genes

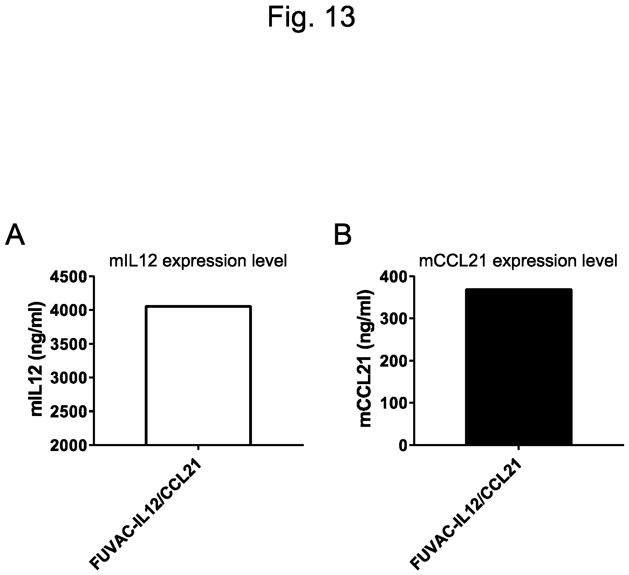

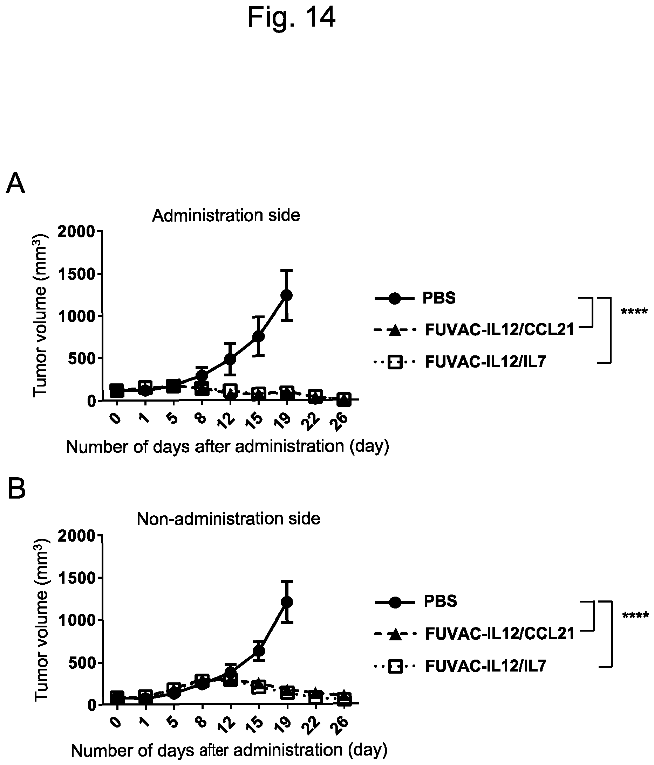

Recombinant vaccinia viruses each carrying and expressing two types of immune regulating genes IL12 and CCL21 or IL12 and IL7 (FUVAC-IL12/CCL21 or FUVAC-IL12/IL7) were prepared (Figure 12). A549 cells were seeded on a 96-well plate at 1.0 × 10⁴ cells/well and cultured for 24 hours, and then FUVAC-IL12/CCL21 was allowed to infect A549 cells at MOI = 0.1. After cells were cultured at 37°C for 48 hours, each cell supernatant was collected, and IL12 and CCL21 in the supernatant were measured using Mouse IL12 p40/p70 ELISA Kit (RayBiotech) and Mouse CCL21/6Ckine Quantikine ELISA Kit (R&D System). The results showed that FUVAC-IL12/CCL21 had expressed and produced both IL12 and CCL21 (Figure 13). Figure 13 shows the concentrations of IL12 and CCL21 in the supernatant (n = 1). Subsequently, using the allogeneic transplantation model shown Figure 4 as in Example 3, the treatment effect of FUVAC-IL12/CCL21 and FUVAC-IL12/IL7 was investigated by measuring the tumor diameter after PBS or each of the viruses (2.5 × 10⁷ PFU) was directly administered into a tumor on one side three times every other day (Days 0, 2, and 4). The results of a statistical analysis by two-way ANOVA demonstrated that, as compared with administration of PBS, the tumor diameter was significantly reduced on the virus administration side and the non-administration side at 19 days after administration of FUVAC-IL12/CCL21 and FUVAC-IL12/IL7 (Figure 14: ****, p < 0.0001).

From the above, the treatment effect of a combination of two types of FUVACs each carrying and expressing one type of different immune regulating gene was equivalent to the treatment effect of FUVAC carrying and expressing two types of immune regulating genes simultaneously. It was shown that FUVACs each carrying and expressing one type of different immune regulating gene can be used in combination and, in addition, that a FUVAC carrying and expressing two types of immune regulating genes simultaneously can also be used.

### Example 7 Effect of the cell fusion ability of recombinant vaccinia viruses carrying and expressing two types of immune regulating genes

On the basis of the above-described vaccinia virus VGF-LucGFP/O1L-DsRed:MDRVV and transfer vector plasmid DNA pTNshuttle/VGF-SP-mIL12, a recombinant virus was collected by the same method as described above using loss of GFP expression as an indicator and designated as MDRVV-IL12. Subsequently, on the basis of the vaccinia virus MDRVV-IL12 and the transfer vector plasmid DNA pTNshuttle/O1L-SP-mCCL21, the recombinant virus was collected by the same method as described above using loss of DsRed expression as an indicator and designated as MDRVV-IL12/CCL21. Each of recombinant viruses having no problem in the nucleotide sequence was cultured in a large amount in A549 cells and purified by the above-described method, then the viral titer was measured in RK13 cells, and the recombinant viruses were subjected to experiments.

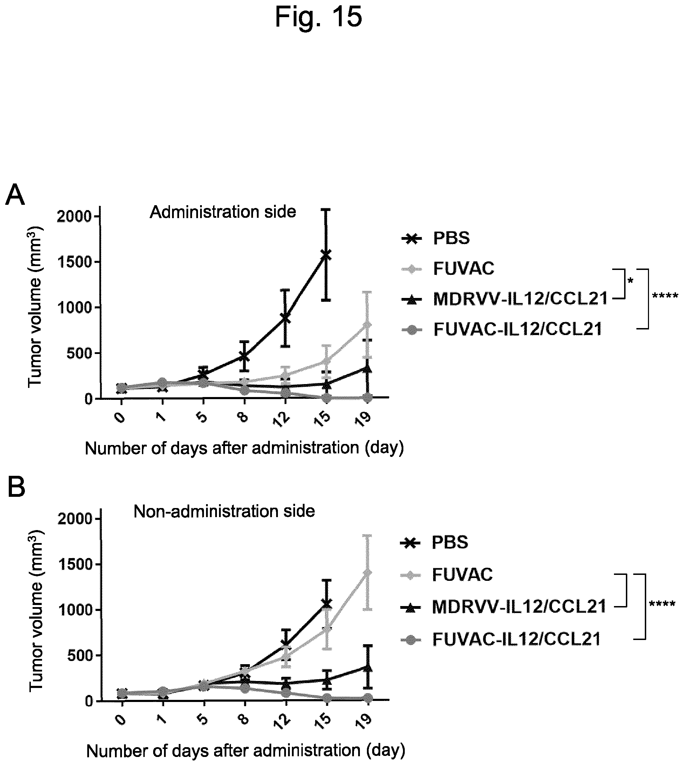

Using the allogeneic transplantation model shown in Figure 4, the treatment effect of FUVAC, MDRVV-IL12/CCL21, and FUVAC-IL12/CCL21 was investigated by measuring the tumor diameter after a single dose of PBS or each of the viruses (5 × 10⁷ PFU) was administered directly into a tumor on one side (Day 0). The results of a statistical analysis by two-way ANOVA demonstrated that, as compared with administration of FUVAC, the tumor diameter was significantly reduced on the virus administration side and the non-administration side at 19 days after administration of MDRVV-IL12/CCL21 or FUVAC-IL12/CCL21 (Figure 15: *, p < 0.05; ****, p < 0.0001).

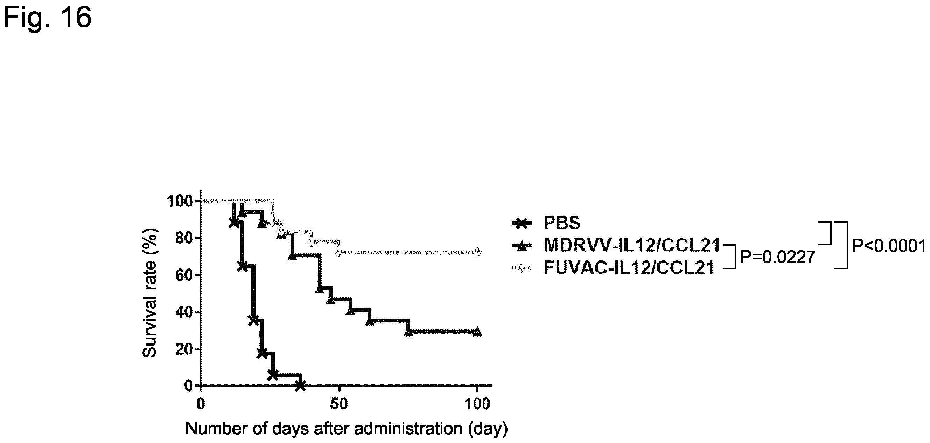

Subsequently, in a similar allogeneic transplantation model, the survival time was assessed after a single dose of PBS, MDRVV-IL12/CCL21 (5 × 10⁷ PFU), or FUVAC-IL12/CCL21 (5 × 10⁷ PFU) was administered directly into a tumor on one side (Day 0). The results of a statistical analysis by the log-rank test showed that the survival time was significantly prolonged in mice given MDRVV-IL12/CCL21 or FUVAC-IL12/CCL21 virus as compared with in mice given PBS. Further, FUVAC-IL12/CCL21 significantly prolonged the survival time as compared with MDRVV-IL12/CCL21 (Figure 16). It is notable that complete remission of tumor was achieved on both sides in 13 of the 18 mice given FUVAC-IL12/CCL21 and five of the 17 mice given MDRVV-IL12/CCL21 (Table 1).

**[Table 1]**

| | Administration side | Non-administration side | Both sides |
|---|---|---|---|
| PBS | 0/17 | 0/17 | 0/17 |
| MDRVV-IL12/CCL21 | 14/17 | 5/17 | 5/17 |
| FUVAC-IL12/CCL21 | 17/18 | 13/18 | 13/18 |

From the above, when even a vaccinia virus not having a cell fusion ability (MDRVV) carries and expresses two types of immune regulating genes (IL12 and CCL21) simultaneously, a single dose of the virus exhibited a very high treatment effect not only on the virus administration side but also on the virus non-administration side, thus prolonging the survival time. Further, when a cell fusion ability is added to a vaccinia virus in addition to these two types of immune regulating genes, that is, when IL12 and CCL21 are simultaneously carried and expressed in a vaccinia virus having a cell fusion ability (FUVAC), the anticancer effect thereof is enhanced to an extent that cannot be expected, thus prolonging the survival time.

### Example 8 Treatment effect of a combination of three types of immune regulating genes carried and expressed in recombinant vaccinia viruses

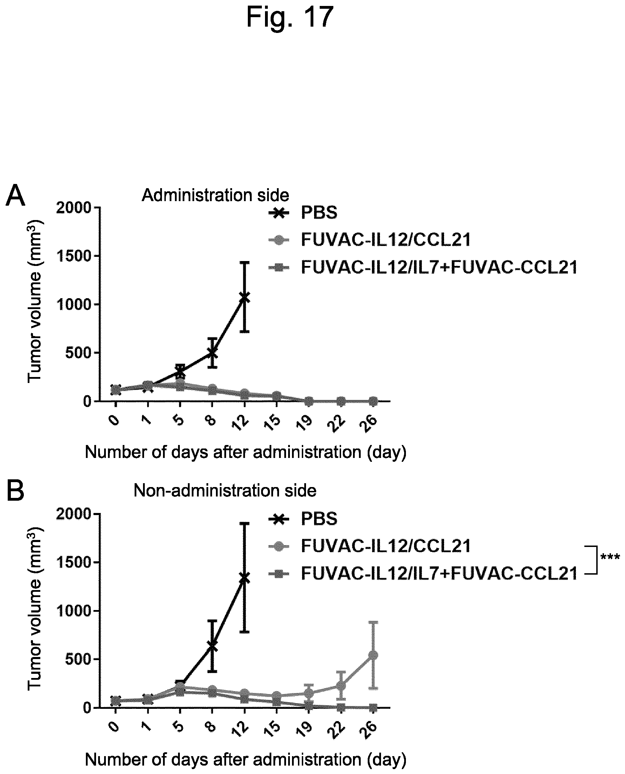

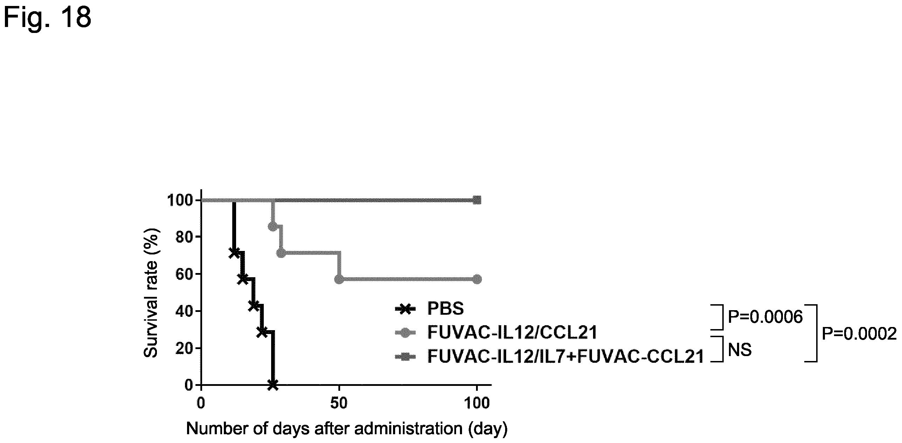

Using the allogeneic transplantation model shown Figure 4 as in Example 7, a single dose of PBS or each of the viruses was directly administered into a tumor on one side (Day 0). The treatment effect of the combination of viruses was investigated by measuring the tumor diameter and examining the survival curve. The results of a statistical analysis by two-way ANOVA demonstrated that, as compared with administration of FUVAC-IL12/CCL21, there was no difference in the tumor diameter on the virus administration side at 26 days after administration of a combination of FUVAC-IL12/IL7 and FUVAC-CCL21, but the tumor diameter was significantly reduced on the non-administration side (Figure 17: ***, p < 0.001). A statistical analysis by the log-rank test demonstrated that, although there was no significant difference in prolongation of the survival time between FUVAC-IL12/CCL21 and a combination of FUVAC-IL12/IL7 and FUVAC-CCL21, both thereof significantly prolonged the survival time as compared with that in mice given PBS (Figure 18). It is notable that complete remission of the tumor was achieved on both sides in seven of the seven mice given a combination of FUVAC-IL12/IL7 and FUVAC-CCL21 and four of the seven mice given FUVAC-IL12/CCL21 (Table 2).

**[Table 2]**

| | Administration side | Non-administration side | Both sides |
|---|---|---|---|
| PBS | 0/7 | 0/7 | 0/7 |
| FUVAC-IL12/CCL21 | 7/7 | 4/7 | 4/7 |
| FUVAC-IL12/IL7+FUVAC-CCL21 | 7/7 | 7/7 | 7/7 |

From the above results, the treatment by a combination of three types of immune regulating genes, even single administration of the viruses, exhibited a very high treatment effect that cannot be expected not only on the administration side but also on the non-administration side, thus prolonging the survival time.

### Industrial Applicability

The vaccinia virus of the present invention can be used for cancer treatment.

### Sequence listing free text

### SEQ ID NOS: 1 to 4, 10 to 13 primers

All publications, patents, and patent applications cited in the present specification are incorporated into the present specification by reference as they are.

## Claims

1. A vaccinia virus comprising at least one immune regulating gene as an exogenous gene.

2. The vaccinia virus according to claim 1, which comprises two or three immune regulating genes as exogenous genes.

3. The vaccinia virus according to claim 1 or 2, wherein the vaccinia virus is the LC16 strain, the LC16mO strain, or the LC16m8 strain modified so that the B5R gene is expressed.

4. The vaccinia virus according to any one of claims 1 to 3, which is deficient in functions of the K2L gene or the HA gene or the K2L gene and the HA gene and induces cell death by causing fusion of infected cells.

5. The vaccinia virus according to any one of claims 1 to 4, which is an oncolytic vaccinia virus.

6. The vaccinia virus according to claim 4 or 5, which does not grow in a normal cell, but grows specifically in a cancer cell and has an oncolytic effect damaging a cancer cell specifically.

7. The vaccinia virus according to any one of claims 1 to 6, wherein the immune regulating gene is selected from the group consisting of the gene encoding IL-12, the gene encoding CCL21, the gene encoding IL-7, the gene encoding PDlscFv (scFv of anti-PD-1 antibody), and the gene encoding CD40L (CD154).

8. The vaccinia virus according to any one of claims 1 to 7, which comprises the gene encoding IL-12 and the gene encoding CCL21 in combination.

9. The vaccinia virus according to any one of claims 1 to 7, which comprises the gene encoding IL-12, the gene encoding CCL21, and the gene encoding IL-7 in combination.

10. The vaccinia virus according to any one of claims 1 to 7, which comprises any gene combination of the following (I) to (v):
(I) a combination of the gene encoding IL-12 and the gene encoding IL-7;
(ii) a combination of the gene encoding IL-12 and the gene encoding PD1scFv;
(iii) a combination of the gene encoding IL-7 and the gene encoding CCL21;
(iv) a combination of the gene encoding CD40L and the gene encoding IL-7; and
(v) a combination of the gene encoding CD40L and the gene encoding CCL21.

11. A pharmaceutical composition for cancer treatment which comprises the vaccinia virus according to any one of claims 1 to 10.

12. A composition comprising vaccinia viruses in combination, the vaccinia viruses being two or more types of the vaccinia virus according to any one of claims 1 to 10 comprising one or two immune regulating genes as exogenous gene(s), wherein each of the vaccinia viruses comprises different immune regulating gene(s).

13. The composition according to claim 12, which comprises in combination a vaccinia virus comprising a combination of the gene encoding IL-12 and the gene encoding IL-7 and a vaccinia virus comprising the gene encoding CCL21.

14. The composition according to claim 12 or 13, which is a pharmaceutical composition for cancer treatment.

15. A kit comprising vaccinia viruses in combination, the vaccinia viruses being two or more types of the vaccinia virus according to any one of claims 1 to 10 comprising one or two immune regulating genes as exogenous gene(s), wherein each of the vaccinia viruses comprises different immune regulating gene(s).

16. The kit according to claim 15, which comprises in combination a vaccinia virus comprising a combination of the gene encoding IL-12 and the gene encoding IL-7 and a vaccinia virus comprising the gene encoding CCL21.

17. The kit according to claim 15 or 16, which is a kit for cancer treatment.
